# EUROPEAN PATENT APPLICATION

(11) **EP 1 514 551 A1**
(43) Date of publication of application: **16.03.2005**
(21) Application number: 03733176.6
(22) Date of filing: 29.05.2003
(51) Int. Cl.: A61K 31/702, A61K 31/715, A61P 11/06, A61P 29/00, A61P 37/08, A61P 43/00, A23L 1/30, C08B 37/00, C07H 3/06, C07H 3/04

(54) **ANTIINFLAMMATORY AGENT, AGENT FOR PREVENTING/AMELIORATING ALLERGIC DISEASES AND FUNCTIONAL FOOD**

(30) Priority: 31.05.2002 JP 2002159158
(71) Applicant: Amano Enzyme Inc., Nagoya-shi, Aichi 460-8630 (JP); Bio Research Corporation of Yokohama, Yokohama-shi, Kanagawa 236-0004 (JP)
(72) Inventor: HASHIMOTO, Hiroyuki, Shimamoto-cho, Mishima-gun, Osaka 618-00 (JP); SONOYAMA, Kei, Toyohira-ku, Sappori-shi, Hokkaido 062-0 (JP); FUJITA, K. BIO RESEARCH CORP.YOKOHAMA Yokohama-Kan, Kanazawa-ku Yokohamashi,Kanagawa236-0004 (JP); HARA, k. BIO RESEARCH CORP.YOKOHAMA Yokohama-Kan, Kanazawa-ku Yokohamashi,Kanagawa236-0004 (JP); OKADA, Masamichi AMANO ENZYME INC.GIFU R & D CNTR, Kakamigahara-shi, Gifu 509-0108 (JP); MORI, Shigeharu AMANO ENZYME INC.GIFU R & D CNTR, Kakamigahara-shi, Gifu 509-0108 (JP)
(74) Representative: Beacham, Annabel Rose
(86) International application number: PCT/JP2003/006783
(87) International publication number: WO 2003/101464

(57) **Abstract**

It is intended to provide an antiinflammatory agent made from a highly safe material which can be daily taken as a food, an agent for preventing or ameliorating allergic diseases and a functional food having the above effects. The antiinflammatory agent contains an α-bond galactooligosaccharide as the active ingredient. The agent for preventing or ameliorating allergic diseases contains an α-bond galactooligosaccharide as the active ingredient. The functional food contains an α-bond galactooligosaccharide as the active ingredient. As such α-bond galactooligosaccharide, use can be made of a product synthesized by the dehydration reaction by α-galactosidase with the use of galactose or a galactose-containing material as a substrate.

## Description

### Technological Field

The present invention relates to an anti-inflammatory agent containing an α-bond galactooligosaccharide as the active ingredient, an agent for preventing or ameliorating allergic diseases and a functional food containing an α-bond galactooligosaccharide as the active ingredient and having an anti-inflammatory action or an action for preventing or ameliorating allergic diseases.

### Background Technology

Inflammation means a series of reactions shown by an organism against any internal or external stimulations, and includes inflammation due to infection by bacterium, virus, parasite and the like, inflammation by heat, cooling, mechanical trauma, ultraviolet ray and the like, allergic inflammation caused by deposition of an immune complex produced in a body on cells and tissue, inflammation due to an abnormal metabolic product generated in a body, and the like, and the inflammations invite pyrexia, flare, swelling, pain, dysfunction and the like. For treatment of such inflammations, there are used steroidal anti-inflammatory agents, non-steroidal anti-inflammatory agents, anti-phlogistic enzymes, anti-histaminic agents and the like.

Now, there is a tendency of increase in the number of patients suffering from allergic diseases such as pollenosis, food allergy, atopic dermatitis, allergic rhinitis, anaphylaxis, allergic bronchial asthma and the like. Allergy is a reaction causing disorders in an organism as a result of an immune response, and the above-mentioned allergic diseases are classified into type I allergy correlated with an IgE antibody produced by a B cell. In type I allergy, an IgE antibody produced by stimulation by an antigen such as microorganisms, food and the like bonds to a mastocyte (mast cell), and the same antigen bonds again to this, consequently, various chemical mediators such as histamine, leucotriene, serotonin, prostaglandin and the like are secreted, to cause accentuation of vascular permeability, bronchoconstriction, tissue damage and the like (immediate phase). In allergic bronchial asthma and the like, it is becoming clear that tissue damage of bronchial mucous membrane, and the like are caused by remarkable invasion of eosinophils (late phase).

On the other hand, according to the immune mechanism described above, proteins and the like, among food components taken and incorporated into an organism, act as a so-called antigen causing an immune response, and this antigen is taken to cause a supersensitive immune reaction.

However, an organism has a control mechanism of preventing occurrence of a supersensitive immune response such as allergy and the like against a food antigen referred to as oral immune tolerance. This oral immune tolerance is induced by rendering a specific T cell against a food antigen into nonresponse condition (anagy), or causing apoptosis (cell death) to allow the cell disappear, and when the mechanism of oral immune tolerance is broken for some reasons, an IgE antibody is produced against certain kind of taken food to cause food allergy, in some cases.

The above-mentioned IgE antibody inviting type I allergy is produced by activation of B cells by IL-4 and the like secreted from differentiated and induced Th2, among two different subsets differentiated from helper T cells (Th0) (namely, type 1 helper T cell (differentiated and induced from interleukin 12 (IL-12) and the like, hereinafter referred to as Th1) and type 2 helper T cell (differentiated and induced from interleukin 4 (IL-4) and the like, hereinafter referred to as Th2)). Th1 and Th2 are in a correlation suppressing mutual proliferation via a cytokine, and the immune response is originally present under a mechanism of mutual control of both the cells to make a balance. Therefore, type I allergy is believed to be a result of deviation of balance toward Th2 because of some reasons, and it is believed that if this deviation is corrected, in other words, if Th1 is made dominant, prevention and amelioration of allergic diseases is possible. There is also an idea that increase in the number of patients suffering from allergic diseases is ascribable to dominance of Th2 owing not only to gene factors but also to environmental factors (decrease in chances of invasion of a microorganism into a body due to improvement in hygienic condition, increase in chances of exposure to chemical substance, increase in stress, and the like), that is, prevention and amelioration of allergic diseases is a current important subject.

However, conventionally, therapeutic agents for inflammation and therapeutic agents for allergic diseases are mostly composed of medical drugs, and when a treatment for a long period of time is necessary such as in the case of chronic inflammation, atopic dermatitis, allergic bronchial asthma and the like, some side effects follow in chronic dosing, and a treatment showing high safety even in chronic dosing is desired. Further, recently, there is a tendency also of increase in the number of patients suffering from food allergy against various foods, and a highly safe treatment is desired. Furthermore, medical drugs used for the treatment of allergic diseases are mostly composed of those used symptomatically such as anti-histaminic agents, and a causal treatment is desired.

The present invention has been made in view of the above-mentioned facts and provides an anti-inflammatory agent made from a highly safe material which can be daily taken as a food, an agent for preventing or ameliorating allergic diseases depending on the mechanism of onset of allergic diseases, and a functional food having these actions.

### DISCLOSURE OF THE INVENTION

The present invention is an anti-inflammatory agent containing an α-bond galactooligosaccharide as the active ingredient.

The present invention is an agent for preventing or ameliorating allergic diseases, containing an α-bond galactooligosaccharide as the active ingredient. In this agent for preventing or ameliorating allergic diseases, prevention or amelioration of allergic diseases accentuates the immune response of Th1 and suppresses the immune response of Th2. An α-bond galactooligosaccharide accentuates Th1 and suppresses Th2 and adjusts a balance of them, therefore, this saccharide can suppress production of an IgE antibody. In this agent for preventing or ameliorating allergic diseases, prevention or amelioration of allergic diseases suppresses production of an IgE antibody and invasion of an eosinophile. Since an α-bond galactooligosaccharide strongly suppresses invasion of an eosinophile, it is useful for allergic bronchial asthma in which an allergic reaction is caused in a late phase. Also, an α-bond galactooligosaccharide promotes induction of oral immune tolerance, and consequently, is useful for food allergy.

The present invention is a functional food containing an α-bond galactooligosaccharide as the active ingredient and having an anti-inflammatory action or an action for preventing or ameliorating allergic diseases.

The α-bond galactooligosaccharide is an oligosaccharide having an α-galactosyl group, and includes melibiose, manninotriose, raffinose, stachyose and the like, and these can be produced from beat, soy bean oligosaccharide and the like. As the α-bond galactooligosaccharide, commercially available products can also be used.

Further, as the α-bond galactooligosaccharide, those synthesized with enzymes can be used. Synthesis with an enzyme can be conducted utilizing the dehydration condensation reaction of α-galactosidase with the use of galactose or galactose-containing substance as a substrate. By synthesis using galactose as a substrate, α-bond galactooligosaccharides generally represented by α-(Gal)ₙ (n usually represents an integer of 2 to 10) including a mixture of α-galactobiose, α-galactotriose, α-galactotetraose, and higher oligosaccharides, are obtained.

By synthesis using galactose and glucose as a substrate, α-bond galactooligosaccharides generally represented by α-(Gal)ₙGlc (n usually represents an integer of 1 to 9) including a mixture of oligosaccharides composed only of galactoses such as α-galactosylglucose, α-galactobiose and the like, are obtained. The galactose-containing substance also includes hydrolysates of galactose-containing substances, for example, a mixture of galactose and glucose obtained by allowing β-galactosydase and acid to act on lactose, and the like. A case in which glucose is mixed separately with galactose and used as a substrate is also included (see WO 02/18614). These α-bond galactooligosaccharides have been confirmed to be safe and can be daily used.

The source of α-galactosidase is not particularly restricted providing it can be used for synthesis of an α-bond galactooligosaccharide by a dehydration condensation reaction with the use of galactose or galactose-containing substance as a substrate, and from the standpoint of yield or the like, those produced by Aspergillus niger are preferable, and those produced by Aspergillus niger APC-9319 strain (FERM BP-7680) are more preferable (see, WO 02/18614).

This strain has been deposited with National Institute of Advanced Industrial Science and Technology International Patent Organism Depositary Center (IPOD, 305-8566, chuo No. 6, higashi 1-1-1, Tsukuba city, Ibaraki prefecture, Japan) (relegated from national deposition to international deposition, original deposition date: August 29, 2000, acceptance No. of national deposition: FERM P-18003) according to the Budapest Treaty.

Mycological natures of this strain are described below.

Conidiospore head: spherical to radial, conidiospore (optical microscope): spherical to sub-spherical, smooth surface to slightly rough surface, conidiospore (electron microscope): rough surface (having elevation), diameter (about 3. 5 to 4.0 µm), effusion: formed in small amount (clear to brown) , smell: scarce, sterigmata: 2 stage, colony growth speed (malt extract agar medium, culturing at 25°C) : >85 mm (7 days culture), >85 mm (12 days culture), colony growth speed (Czapek Yeast agar medium, culturing at 25°C): 60 to 67 mm (7 days culture), 65 to 73 mm (12 days culture), hue of colony (malt extract agar medium) : black (surface), colorless (rear surface), hue of colony (Czapek Yeast agar medium) : black to gray black (front surface) , cream to gray yellow (rear surface)

The effective amount of an α-bond galactooligosaccharide which is an active ingredient of the anti-inflammatory agent or agent for preventing or ameliorating allergic diseases of the present invention can be appropriately controlled depending on symptom, age, body weight, dose mode and the like, and usually, for adult, it is preferably from 4 mg to 40 g/day, more preferably from 0.3 g to 30 g/day. Regarding the functional food, when an α-bond galactooligosaccharide is contained in other food materials, it is preferably taken in a rate of 0.3 g to 30 g/day, and the rate can be appropriately controlled depending on the kind, form, shape and the like of a food utilized.

Though it is general that an α-bond galactooligosaccharide in the form of liquid or powder is orally administered, the dose mode is not limited to this. Namely, the dosage form of an α-bond galactooligosaccharide can be appropriately selected, and it can be processed into various dosage forms such as tablet, powder, granule, capsule, syrup, gargle, ointment and the like using pharmaceutically acceptable various aids such as excipient, binder, disintegrating agent and the like. An effect can also be obtained by injecting an appropriately diluted α-bond galactooligosaccharide aqueous solution into blood.

Further, other medical drugs can be.compounded to give a medical preparation providing the action and effect of an α-bond galactooligosaccharide are not lost.

Also, an α-bond galactooligosaccharide can be compounded in existing suitable foods to give functional foods intending promotion of these functions. For example, an α-bond galactooligosaccharide can be compounded into powdered milk and liquid milk in view of the whole sugar percentage and allowed to be taken.

### BRIEF EXPLANATION OF THE DRAWINGS

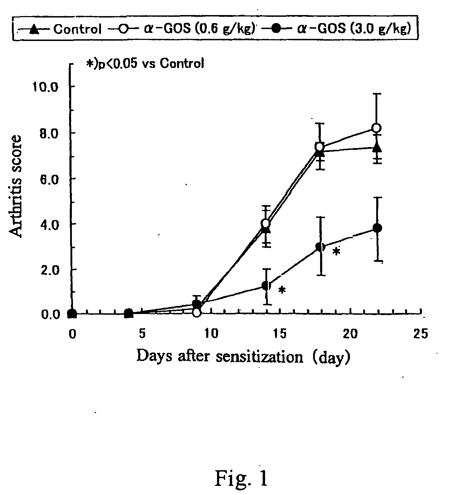

Fig. 1 is a graph showing daily change of arthritis score of rat.
Fig. 2 is a graph showing daily change of leg (right hind leg) volume of rat.
Fig. 3 is a graph showing daily change of leg (left hind leg) volume of rat.
Fig. 4 is a graph showing the relative values of various immune antibodies of rat.
Fig. 5 is a graph showing the number of immunocompetent cells in a bronchovesicle washing liquid of rat.
Fig. 6 is a graph showing change by time of absorbancy in growth of lymphocytes of mouse. In the graph, α-GOS represents ConA stimulation + α-GOS administration, ConA represents ConA stimulation + α-GOS no administration. These definitions are applied also in the following Figs. 7 to 9.
Fig. 7 is a graph showing change by time of absorbancy in growth of lymphocytes of mouse.
Fig. 8 is a graph showing the production amount of IL-12 of mouse.
Fig. 9 is a graph showing the production amount of IL-4 of mouse.
Fig. 10 is a graph showing weekly change of IgG antibody titer exerted by various oligosaccharides.
Fig. 11 is a graph showing weekly change of IgE antibody titer exerted by various oligosaccharides.

### BEST MODES FOR CARRYING OUT THE INVENTION

The present invention will be illustrated further in detail by examples below.

The α-bond galactooligosaccharide used in the examples (hereinafter, referred to as α-GOS, or GOS) had been synthesized by allowing α-galactosidase produced by Aspergillus niger APC-9319 (FERM BP-7680) to act on galactose, and represented by α-(Gal)ₙ strain (n usually represents an integer of 2 to 10) . Example 1 (effect of preventing onset of adjuvant arthritis)

### 1. Experiment material and method

### (1) Test substance and its preparation method

Suitable amount of α-GOS was weighed, pulverized in a mortar, then, injection distilled water was added to this to cause dissolution thereof, preparing a 600 mg/mL solution. A part of this solution was separated and diluted with injection distilled water to prepare a 100 mg/mL solution. Preparation of a test substance was conducted every one week. The dose volume for rat was 0.6 mL/100 g body weight.

### (2) Preparation of sensitized adjuvant

Suitable amount of Mycobacterium tuberculosis H37Ra (manufactured by Wako Pure Chemical Industries Ltd.) killed by heating was weighed, pulverized in an agate mortar, then, a liquid paraffin (manufactured by Wako Pure Chemical Industries Ltd.) was added portion-wise to cause suspension, preparing a 6 mg/mL suspension.

### (3) Experiment animal

Male Wistar rats (SPF) purchased at 7-weeks old from Nippon SLC K.K. were pre-bred for 11 days and subjected to an experiment. Rats were bred in a SPF barrier breeding room at room temperature 24±3°C and a relative humidity of 55±15% (irradiation time: 8 to 18 hours, ventilation frequency: 18/hour).

Rats were bred at a rate of 2 to 3/cage, and allowed to freely take solid feed (MF, manufactured by Oriental Yeast Industry K.K.) and sterilized distilled water. Picric acid was used in hair for identification of rat individual.

### (4) Induction of arthritis

Rats were fixed on a fixing table under ether anesthesia, and 0.1 mL of the prepared adjuvant was injected intradermally into footpad of right rear leg, to induce arthritis. The induced day was day 0.

### (5) Administration method and group constitution

Administration method: It was orally administered forcibly using a rat oral sonde (administration once per day).
Administration period: day 0 to 21 (here, excepting day 6, 13, 20)
Animal group; 5/group
Group constitution: as shown in Table 1

**Table 1**

| Group No. | Name of substance | Dosage (g/kg) | Individual No. |
|---|---|---|---|
| 1 | Control (distilled water) | 0 | 1-1, 1-2, 1-3, 1-4, 1-5 |
| 2 | α-GOS | 0.6 | 2-1, 2-2, 2-3, 2-4, 2-5 |
| 3 | α-GOS | 3.0 | 3-1, 3-2, 3-3, 3-4, 3-5 |

### (6) Observation and inspection item

General condition: Condition was observed once a day.

Arthritis score: Extents of flare, swelling and rigidity of right front leg, left front leg and left hind leg excepting right hind leg of sensitized portions were visually observed and evaluated by scores of 0 to 4 points according to the following standard, the maximum sum being 12 points. The measurement was conducted on day 0, 4, 9, 14, 18 and 22.
0: no symptom
1: flare and swelling in only one small joint of fingers of four limbs, and the like
2: flare and swelling in two or more small joints or a relatively large joint such as writs and ankle
3: flare and swelling in whole of one hand and leg
4: further, whole swelling of one hand and leg is judged to be maximum (including rigidity of joint)

Leg volume: volume of right hind leg and left hind leg was measured using a leg volume measuring apparatus (TK-105, manufactured by Muromachi Machine). The measurement was conducted on the same day as the evaluation days of arthritis score.

### (7) Statistical treatment

The result arthritis score and leg volume were represented by average value ± standard deviation of individual group. For testing statistical significance of individual group against negative control group, variance analysis (ANOVA) was conducted using an analysis soft (Stat View, Abacus Inc., USA), to confirm equal variance, then, Fisher's PLSD method, multiple comparison test was carried out, to compare between groups. When p<0.05, the statistical significance was positive.

### 2. Experiment result

### (1) Arthritis score

The experiment results are shown in Table 1.

The α-GOS (0.6 g/kg) dose group did not affect arthritis score.

However, the α-GOS (3 g/kg) dose group suppressed remarkably increase in score at day 14 or later. Particularly, at day 14, the score of the control group was 3.8±0.8, while the score of the α-GOS (3 g/kg) dose group was 1.2±0.8 (p<0.05). At day 18, the score of the control group was 7.2±0.4, while the score of the α-GOS (3 g/kg) dose group was 3.0±1.3 (p<0.05).

### (2) Leg volume (right sensitized hind leg)

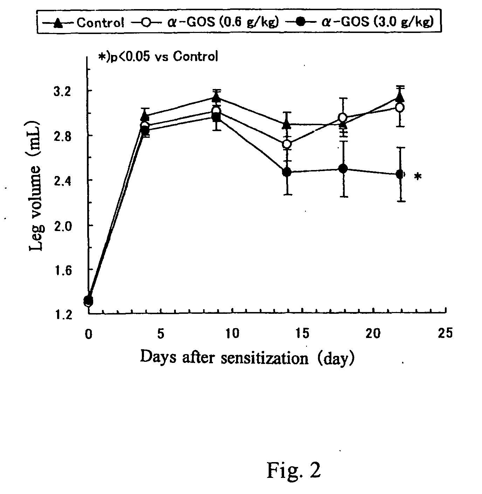

The experiment results are shown in Fig. 2. Like in the case of arthritis score, the α-GOS (0.6 g/kg) dose group did not affect the right leg volume.

However, the α-GOS (3 g/kg) dose group suppressed remarkably increase in right leg volume at day 14 or later. Particularly, at day 22, the volume of the control group was 3.13±0.10 mL, while the score of the α-GOS (3 g/kg) dose group was 2.44±0.24 mL (p<0.05).

### (3) Leg volume (left hind leg)

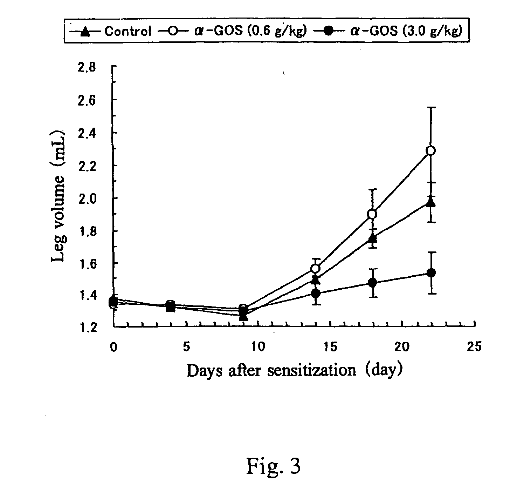

The experiment results are shown in Fig. 3. Like in the case of arthritis score, the α-GOS (0.6 g/kg) dose group did not affect the left leg volume.

While the α-GOS (3 g/kg) dose group did not show significant difference, it showed a tendency of suppressing increase in volume at day 14 or later.

### 3. Consideration

It is suggested that by forced oral administration of α-GOS (3 g/kg), a remarkable suppressing effect on adjuvant arthritis of rat is manifested, and particularly, an anti-inflammatory effect against chronic inflammation is expected.

### Example 2 (effect of preventing allergic bronchial asthma)

### 1. Experiment material and method

Using Brown Norway rat (BN rat) of which response of an antibody against an administered antigen has been reported to be near that of human, an allergic bronchial asthma model was produced using ovalbumin (OVA) as a model antigen, and an influence exerted by α-GOS added to feed was analyzed. Until initiation of the experiment, rats were allowed to freely take AIN-93G as a base feed (α-corn starch, 529.5 g/kg; casein, 200 g/kg; sucrose, 100 g/kg; soy bean oil, 70 g/kg; cellulose, 50 g/kg; mineral mixing, 35 g/kg; vitaminmixing, 10 g/kg; L-cystine, 3 g/kg; choline bitartarate, 2.5 g/kg). After pre-breeding for 1 week, 1 mL of mixed liquid of OVA (1 mg/mL) and alum was subcutaneously administered to a cervical back part. Simultaneously, inactivated Bordetella pertussis (6×10⁹ CFU/rat) was intraperitoneally administered as an adjuvant. One week after immunization, a base feed group and an α-GOS added feed group of giving a feed in which 5% (w/w) of the base feed is substituted with α-GOS were prepared, and rats were allowed to freely take the test feeds for 2 weeks. During this, OVA (1 mg/rat) was orally administered four times to half individuals of the α-GOS added feed group. 3 weeks after immunization, rats were allowed to absorb aerosol of OVA (1% (w/v)) for 10 minutes using an ultrasonic nebulizer. The day after elicitation, the abdomen was open under Nembutal anesthesia, blood was collected from abdominal aorta to cause bleeding to death, then, bronchovesicule washing liquid (BALF) was obtained. A site spin specimen was made from BALF, May-Grunwald-Giemsa stain was performed and cell profile was analyzed. The OVA specific antibody level in blood was measured by ELISA.

### 2. Experimental result

It is becoming clear that the pathological condition of allergic bronchial asthma is composed of an immediate phase correlated with degranulation of a mast cell by antigen-specific IgE and a late phase composed mainly of the subsequently eosinophilic inflammation, therefore, also in this example, the OVA specific antibody level in blood and the extent of eosinophile invasion in an air duct after antigen elicitation were used as indices.

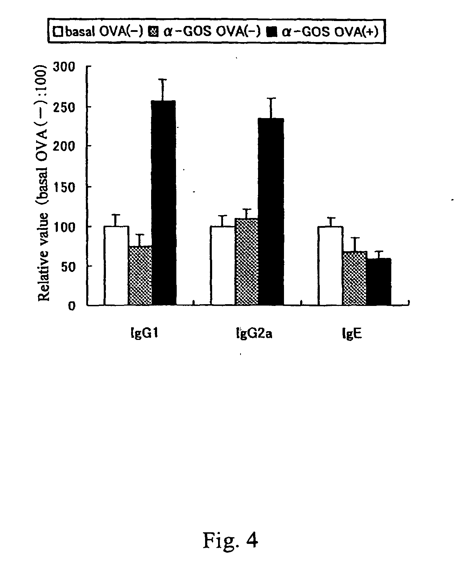

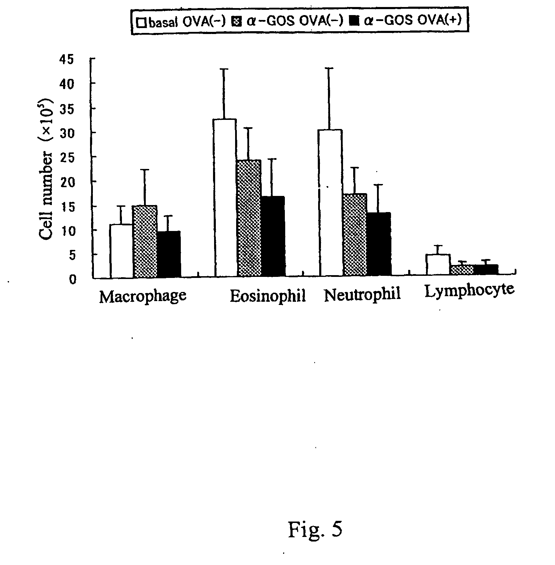

Regarding the antigen-specific IgE level in blood, there is a tendency that this level is lower in the α-GOS added group than in the base feed group as a control group (Fig. 4). On the other hand, there is a tendency that the number of eosinophiles and the number of neutrophiles in BALF are smaller in the α-GOS added group than in the base feed group, further, it is about half in the case of oral administration of OVA (Fig. 5).

From the above-mentioned results, it is clear that α-GOS has an effect for preventing allergic asthma in an allergic asthma model.

When OVA is orally administered after immunization, a further lower value is shown as compared with the case of no oral administration. The reason for this is hypothesized that an action of suppressing a symptom is manifested by promotion of induction of oral immune tolerance due to OVA by a feed α-GOS. From the above-mentioned results, it is clear that α-GOS accentuates induction of oral immune tolerance, and is effective also for prevention and reduction of food allergy.

### Example 3 (lymphocyte growth effect)

### 1. Experiment material and method

### (1) Animal

5 weeks C57BL/6J Jcl female mice were purchased from Clea Japan, Inc. Until the test, mice were allowed to freely take a commercially available feed (CE-2, manufactured by Clea Japan, Inc.) and tap water, and bred under environments of a temperature of 24±1°C, a humidity of 50±10% and a bright and dark 12-hours cycle (bright period 8:00 to 20:00, dark period 20:00 to 8:00). When used in the test, they were 6-weeks old (body weight, 18.6 g, 20.3 g).

### (2) Mitogen

Concanavalin A (ConA) type IV (manufactured by Sigma) was used as a T cell mitogen.

### (3) Cell culture

C57BL/6 mice were narcotized, by Nembutal (40 mg/kg), and spleen was removed aseptically. The spleen was washed in one shale containing Hank's balanced salt solution to remove fat and the like, and the spleen was ground by a tea strainer and glass bar in the second shale containing the same solution, to prepare float cells. The cells were centrifugally separated at 1500 rpm for 3 minutes, then, the supernatant was removed and the cells were floated in a medium prepared by adding Hepes, L-glutamine, penicillin G, streptomycin, 2-mercaptoethanol and 10% Nuserum (Becton Dickinson Lab.) to a RPMI-1640 medium (manufactured by Nippon Pharmaceutical Co., Ltd.) and centrifugally separated at 1500 rpm for 3 minutes, then, the supernatant was again removed. The cells were floated in a small amount of the same medium, and diluted to 2.5×10⁶ cell/mL. The addition concentration of saccharides was 0 to 1000 µg/mL, ConA was 2 µg/mL, and after preparation, each 200 µL of cells were inoculated onto a 96-well plate, and culturing was initiated in a CO₂ incubator (37°C, CO₂; 5.0%).

### (4) Cell growth measurement

0, 24, 48 and 72 hours after culturing, cell growth was measured by a MTT method. 0.5% MTT (3-(4,5-dimethyl-2-thiazolyl)-2,5-diphenyl-2H-tetrazolium bromide) solution (dissolved in RPMI-1640 medium) was added to each well in an amount of 10 µL, and reacted for 3 hours in a CO₂ incubator (37°C, CO₂; 5.0%). A SDS solution (20% SDS in 0.03 NHCl) was added to each well in an amount of 80 µL, to terminate the reaction. It was allowed to stand for about 10 hours to solubilize a coloring matter, then, the absorbancy at 540 nm was measured by a micro plate reader. Cell growth was evaluated by absorbancy difference (ΔOD) obtained by subtracting blank absorbancy from the absorbancy in the culture liquid.

### (5) Measurement of cytokine

Production of cytokines was measured using the samples under α-GOS 125 and 500 µg/mL addition conditions in the lymphocyte growth ability evaluation test. 72 hours after initiation of culturing, the plate was frozen to terminate culture. Cytokines (LL-4, IL-12) in each culture liquid were measured by an ELISA kit (manufactured by Amersham Pharmacia).

### 2. Experimental results

### (1) Test for evaluation of lymphocyte growth ability of α-GOS

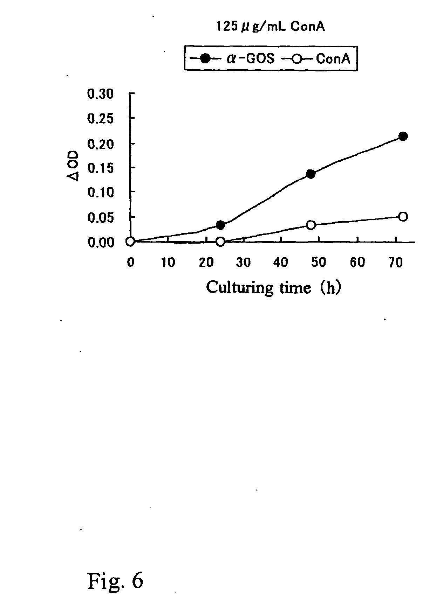

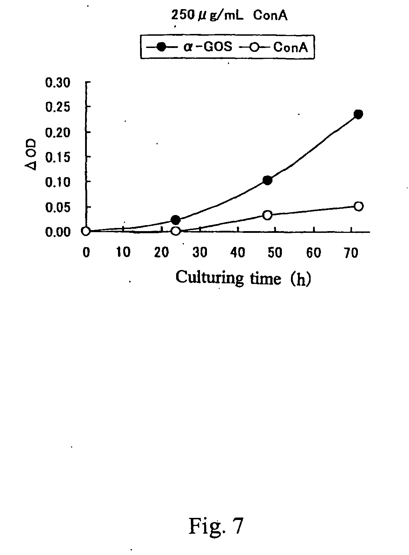

The prepared float cell liquid showed a concentration of 2.3×10⁶ cell/mL and a survival rate of 96%. Change in absorbancy difference (ΔOD) following lymphocyte growth at each saccharide concentration is shown in Fig. 6 and Fig. 7.

48 hours or later after initiation of culturing, cell growth by ConA was observed in the α-GOS added group.

### (2) Measurement of cytokine production of α-GOS

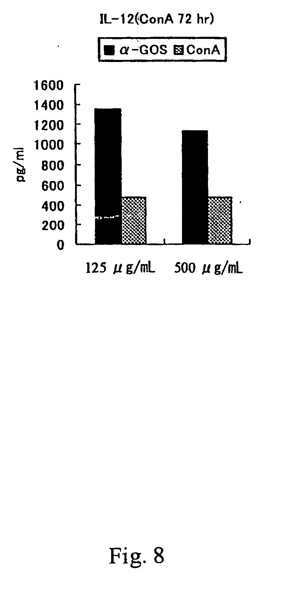

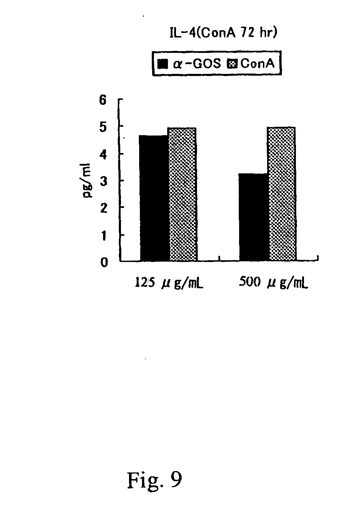

The results of measurement of production of IL-4 and IL-12 when α-GOS and ConA were added to a plate are shown in Fig. 8 and Fig. 9.

IL-12 production in stimulating with ConA (after 72 hours) showed a value twice or more than those in no-addition section both in the cases of α-GOS 125 µg/ml and α-GOS 500 µg/ml. In contrast, IL-4 production showed approximately the same amount as that of no-addition section in the case of addition of α-GOS 125 µg/ml, while a lower value was shown than that in no-addition section in the case of addition of 500 µg/ml.

From the above-mentioned results, it is clear that α-GOS has an action of growing a T cell, by addition of a mitogen (ConA) . In this case, α-GOS accentuates IL-12 production and suppresses IL-4 production. Namely, it becomes clear that α-GOS accentuates an immune response of Th1 and suppresses an immune response of Th2.

### Example 4 (influence exerted on allergic bronchial eosinophilic inflammation by oligosaccharide)

### 1. Experimental method

An oral sensitization model of BALB/c mouse using ovalbumin (OVA) as a model antigen was produced, and influences exerted by various oligosaccharides added to feed on serum antibody titer were compared. Until initiation of the experiment, 12-week old male BALM/c mice were allowed to freely take AIN-93G as a base feed (α-corn starch, 529.5 g/kg; casein, 200 g/kg; sucrose, 100 g/kg; soy bean oil, 70 g/kg; cellulose, 50 g/kg; mineral mixing, 35 g/kg; vitamin mixing, 10 g/kg; L-cystine, 3 g/kg; choline bitartarate, 2.5 g/kg). After pre-breeding for 1 week, an oligosaccahide no-addition seed (base seed) group, and feeds obtained by substituting 5% (w/w) of the base feed with raffinose (RAF), α-GOS (hereinafter, referred to simply as GOS), fructooligosaccharide (FOS), xylooligosaccharide (XOS), that is, five groups were prepared, and mice were allowed to freely take these feeds for 8 weeks. During this, an aqueous OVA solution (1 mg/0.2 mL) was orally administered forcibly every day. After initiation of aministration of OVA, blood was collected from orbital venous plexus every one week, and centrifugally separated to obtain sera. Sear of one group (9/group) were pooled, and subjected to quantification of antigen-specific antibody titer by ELISA.

### 2. Result and consideration

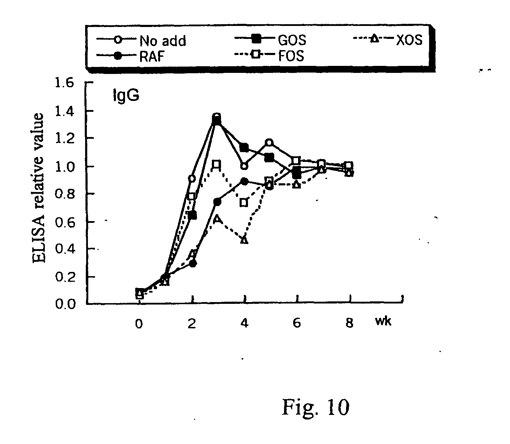

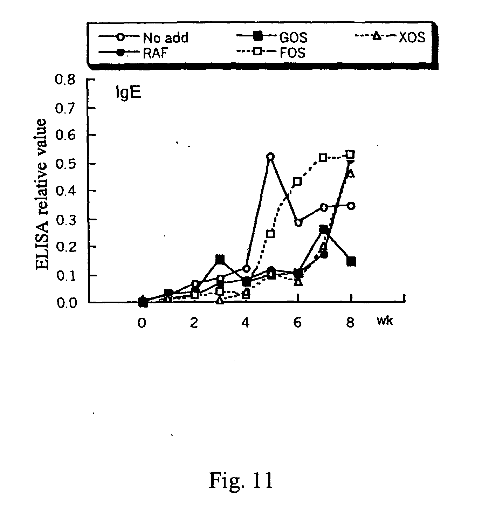

Changes in concentration of antigen-specific IgG and IgE in blood from initiation of oral immunization of OVA to 8-weeks are shown in Fig. 10 and Fig. 11. Before administration of OVA (0 week), both IgG and IgE were not found.

IgG was detected from one week after initiation of administration of OVA, in the oligosaccharide no-addition feed group, a plateau was attained in three weeks, after this, until 8 weeks, approximately the same level was maintained. The GOS feed group showed the same change as that for the oligosaccharide no-addition feed group throughout the experiment period, while both the FOS feed group and the RAF and XOS feed groups changed lower than the oligosaccharide no-addition feed group, at 3 to 4 weeks and at 2 to 5 weeks, respectively. However, at 6 weeks, all the groups converged to approximately the same level.

On the other hand, IgE showed slow general increase as compared with IgG. In the oligosaccharide no-addition feed group, the value initiated to increase from 2 weeks, and showed higher values from the temporary maximum value at 5 weeks to high values at 8 weeks. In contrast, the FOS feed group showed an increase at 5 weeks, thereafter, continued an increase until 8 weeks. The RAF and XOS feed groups changed lower than the oligosaccharide no-addition feed group and the FOS feed group until 7 weeks, and showed the same high value as that of the FOS group. The GOS feed group changed lower like the RAF and XOS feed groups until 7 weeks, further, did not increase to the levels of other groups even at 8 weeks.

Form the above-described results, it is believed that RAF and XOS have an effect of delaying increase, against IgG and IgE, and the effect of FOS is weaker than them. On the other hand, though GOS showed no effect of delaying increase of IgG, strongly suppressed increase of IgE, differing from RAF and XOS. Currently, a mechanism for suppressing increase in serum antibody titer by an oligosaccharide is not clear. However, sensitization by oral antigen needs permeation of an antigen from an intestinal tract into a body, therefore, a possibility of delay of sensitization by suppression of permeation through an intestinal tract of an antigen in an intestinal tract by an oligosaccharide, especially GOS is hypothesized.

The present invention is not limited to the above-mentioned examples providing it is included in the substantial range of the invention.

### INDUSTRIAL APPLICABILITY

As described above, an anti-inflammatory agent and an agent for preventing or ameliorating allergic diseases, containing an α-bond galactooligosaccharide as the active ingredient can be used safely for a long period of time, and are useful, particularly, for chronic inflammation or allergic bronchial asthma, and food allergy.

A functional food containing an α-bond galactooligosaccharide as the active ingredient and having an anti-inflammatory action or an action for preventing or ameliorating allergic diseases can be compounded in various foods and can be used safely for a long period of time, and is useful, particularly, for chronic inflammation or allergic bronchial asthma, and food allergy.

## Claims

1. An anti-inflammatory agent containing an α-bond galactooligosaccharide as the active ingredient.

2. The anti-inflammatory agent according to Claim 1, wherein the α-bond galactooligosaccharide is a product synthesized by utilizing the dehydration condensation reaction of α-galactosidase with the use of galactose or galactose-containing substance as a substrate.

3. The anti-inflammatory agent according to Claim 2, wherein the α-bond galactooligosaccharide is a product synthesized using galactose as a substrate and represented by α-(Gal)ₙ (Gal: galactose, n usually represents an integer of 2 to 10).

4. The anti-inflammatory agent according to Claim 2, wherein the α-bond galactooligosaccharide is a product synthesized using as a substrate a substance containing glucose in addition to galactose as the galactose-containing substance and represented by α-(Gal)ₙGlc (Glc: glucose, n usually represents an integer of 1 to 9).

5. An agent for preventing or ameliorating allergic diseases, containing an α-bond galactooligosaccharide as the active ingredient.

6. The agent for preventing or ameliorating allergic diseases according to Claim 5, wherein prevention or amelioration of allergic diseases accentuates the immune response of a type 1 helper T cell (Th1) and suppresses the immune response of a type 2 helper T cell (Th2).

7. The agent for preventing or ameliorating allergic diseases according to Claim 5, wherein prevention or amelioration of allergic diseases is caused by suppression of production of an IgE antibody and suppression of invasion of an eosinophile.

8. The agent for preventing or ameliorating allergic diseases according to Claim 7, wherein the allergic disease is allergic bronchial asthma.

9. The agent for preventing or ameliorating allergic diseases according to Claim 5, wherein prevention or amelioration of allergic diseases is caused by promotion of induction of oral immunity tolerance.

10. The agent for preventing or ameliorating allergic diseases according to Claim 9, wherein the allergic disease is food allergy.

11. The agent for preventing or ameliorating allergic diseases according to Claims 5 to 10, wherein the α-bond galactooligosaccharide is a product synthesized by utilizing the dehydration condensation reaction of α-galactosidase with the use of galactose or galactose-containing substance as a substrate.

12. The agent for preventing or ameliorating allergic diseases according to Claim 11, wherein the α-bond galactooligosaccharide is a product synthesized using galactose as a substrate and represented by α-(Gal)ₙ (n usually represents an integer of 2 to 10).

13. The agent for preventing or ameliorating allergic diseases according to Claim 11, wherein the α-bond galactooligosaccharide is a product synthesized using as a substrate a substance containing glucose in addition to galactose as the galactose-containing substance and represented by α-(Gal)ₙGlc (n usually represents an integer of 1 to 9).

14. A functional food containing an α-bond galactooligosaccharide as the active ingredient and having an anti-inflammatory action or an action for preventing or ameliorating allergic diseases.

15. The functional food according to Claim 14, wherein the α-bond galactooligosaccharide is a product synthesized by utilizing the dehydration condensation reaction of α-galactosidase with the use of galactose or galactose-containing substance as a substrate.

16. The functional food according to Claim 15, wherein the α-bond galactooligosaccharide is a product synthesized using galactose as a substrate and represented by α-(Gal)ₙ (n usually represents an integer of 2 to 10).

17. The functional food according to Claim 15, wherein the α-bond galactooligosaccharide is a product synthesized using as a substrate a substance containing glucose in addition to galactose as the galactose-containing substance and represented by α-(Gal)ₙGlc (n usually represents an integer of 1 to 9).
